# EUROPEAN PATENT APPLICATION

(11) **EP 4 813 351 A1**
(43) Date of publication of application: **30.09.2026**
(21) Application number: 26387040.4
(22) Date of filing: 26.03.2026
(51) Int. Cl.: A61C 19/06, A61N 5/06

(54) **FLEXIBLE MOUTHPIECE**

(30) Priority: 25.03.2025 US 202563777582 P
(71) Applicant: Nikinmaa, Sakari, 02630 Espoo (FI)
(72) Inventor: Nikinmaa, Sakari, 02630 Espoo (FI)
(74) Representative: Laine IP Oy

(57) **Abstract**

The disclosure relates to an intraoral mouthpiece. The intraoral mouthpiece comprises a body defining an arch and having a top level and a bottom level, each level having an inner border, an outer border, and a planar surface disposed between the inner and outer border of each level. The intraoral mouthpiece also comprises a plurality of light sources located on or within the body, which are adapted to deliver light to intraoral surfaces of a user when worn. The body further comprises a central rise portion disposed between a first distal end portion and a second distal end portion, and at least one flexibility-enhancing feature to provide flexibility to at least a portion of the body.

## Description

### Field of the Invention

The invention relates to oral health care. In particular, the invention relates to a flexible mouthpiece for antibacterial treatment of intraoral surfaces, such as teeth. The invention also relates to a method for treating an oral condition, and a method for adjusting the fit of an intraoral mouthpiece for an individual.

### Background of the Invention

Oral health can be promoted by affecting bacteria inside the mouth. According to professional dentists, intraoral bacterial cleaning is required in every two months in order to keep the bacterial biofilm in check. Due to expertise and special equipment needed, this has long been difficult for most of the population. Various intraoral devices, e.g., mouthpieces, to provide photodynamic treatment to a user have been developed to reduce bacteria in the user's mouth. While effectiveness and availability has been improved, the mouthpiece must be tailored and properly fitted to the user's mouth for optimum efficiency. Known mouthpieces, however, lack flexibility of use and their overall effectiveness is suboptimal. Accordingly, improved mouthpieces that can be used more universally are needed.

### Summary of the Invention

The invention is defined by the features of the independent claims. Some specific embodiments are defined in the dependent claims.

According to a first aspect of the present invention, there is provided an intraoral mouthpiece. The intraoral mouthpiece comprises a body comprising a heat conducting material, the body defining an arch and having a top level, a bottom level opposite the top level, each level having an inner border and an outer border opposite the inner border, with a planar surface disposed between the inner and outer border of each level. Further, the intraoral mouthpiece comprises a plurality of light sources located on or within the body and adapted to deliver light to intraoral surfaces of a user when worn. The body further comprises a central rise portion disposed between a first distal end portion and a second distal end portion and at least one flexibility-enhancing feature to provide flexibility to at least a portion of the body.

In some embodiments, the at least one flexibility-enhancing feature comprises the central rise portion being formed from a material having a greater degree of flexibility than the first distal end portion and/or the second distal end portion.

In some embodiments, the first distal end portion and/or the second distal end portion comprises one or more materials selected from the group consisting of metals, transparent or translucent thermoplastic or thermosetting materials, silicon-based polymers, polycarbonates, polyacrylates, filled thermoset composites, liquid crystal polymers, polyimides, and the like.

In some embodiments, the central rise portion comprises a material selected from the group consisting of silicone-based elastomers, polyurethane, and thermoplastic elastomer composites.

In some embodiments, the at least one flexibility-enhancing feature comprises the central rise portion having one or more grooves and/or being of a lesser thickness relative to the first distal end portion and/or the second distal end portion.

In some embodiments, the at least one flexibility-enhancing feature comprises a first corrugated section extending from the central rise portion towards or to the first distal end portion, and a second corrugated section extending from the central rise portion towards or to the second distal end portion.

In some embodiments, the at least one flexibility-enhancing feature comprises a thermally softening material disposed on or within the first distal end portion and/or the second distal end portion, wherein the thermally softening material is configured to soften and increase flexibility of the body upon heating.

In some embodiments, the at least one flexibility-enhancing features comprises at least two perforations positioned at opposite sides of the central rise portion, wherein the at least two perforations are configured to allow permanent mechanical deformation of the body.

In some embodiments, the central rise portion is configured to allow an end of the first distal end portion and/or an end of the second distal end portion to flex at least 0.5 mm, 1 mm, 2 mm, 3 mm, 4 mm, or 5 mm relative to a resting position of the end of the first distal end portion and/or of the end of the second distal end portion.

In some embodiments, the top level and the bottom level further comprise edges extending from the inner border and/or outer border.

In some embodiments, at least some of the edges form an angle of 10 to 55 ° either upwards or downwards relative to the planar surface disposed between the inner and outer border of each level.

In some embodiments, at least some of the edges are tilted so as to form a slope towards the planar surface disposed between the inner and outer border of each level.

In some embodiments, the body comprises a heat conducting material having a thermal conductivity of greater than 0.5 W/mK, for example, greater than 1 W/mK, in particular 1.1 to 25 W/mK for polymer materials and 100 to 500 W/mK for metal materials.

In some embodiments, the body and the light sources are at least partly encapsulated by an encasing having the shape of a dental arch.

In some embodiments, the body comprises an extended portion which forms a cooling tray configured to conduct heat away from the arch of the body.

In some embodiments, the light sources are capable of emitting light at an average power density of 30 to 1000 mW/cm², such as 50 to 500 mW/cm², towards the teeth or intraoral tissue of the user.

According to a second aspect, there is provided a method for adjusting the fit of an intraoral mouthpiece, for example, the intraoral mouthpiece of the first aspect, for an individual.

The method comprises selecting a base size of an intraoral mouthpiece based on a size of the individual's dental arch, and adjusting, based on a shape of the individual's dental arch, a form or configuration of the intraoral mouthpiece, such as to achieve effective light distribution and comfort during treatment. The adjusting comprises reforming the intraoral mouthpiece over an adjustable forming jig.

In some embodiments, the intraoral mouthpiece comprises a first distal end portion and a second distal end portion, each end portion having an end, and the reforming comprises moving an end of the first distal end portion and/or an end of the second distal end portion inwards or outwards, thereby modifying an arch of the intraoral mouthpiece.

In some embodiments, the adjusting further comprises heating the intraoral mouthpiece prior to the reforming.

In some embodiments, the method further comprises cooling the intraoral mouthpiece, allowing the intraoral mouthpiece to set in a desired position for the individual.

In accordance with another aspect, there is provided a method of treatment of teeth or oral tissue of a user, comprising providing a mouthpiece as disclosed herein. The method comprises placing the mouthpiece in the user's mouth; supplying the light sources with electric power for generating light; and directing the light towards the user's teeth or intraoral issue to achieve photodynamic treatment of the teeth and/or oral issue.

In accordance with another aspect, there is provided a kit comprising a mouthpiece according to any of the previous claims and an intraoral agent selected from the group consisting of antioxidants, photosensitizers and potentiating agents, and combinations thereof.

In accordance with another aspect, the kit comprises a photosensitizer, and the photosensitizer is selected from the group consisting of Hypericin, curcumin, phenalenone derivatives, Cercosporin, psoralen, xanthotoxin, Angelicin, alpha-Terthienyl, Phenylthepatriyne, THC, Cannabidiol (CBD). Synthetic photosensitizers include the following: RB (Rose Bengal), MB, Porphyrin derivatives, Curcumin derivatives, Methylene Blue, Indocynine Green, Erythosine, Phenalenone derivatives, Fullerene derivatives, Xanthene derivates, optionally together with a pigment.

### Brief Description of the Drawings

Figure 1 depicts in perspective view of an encasing of the mouthpiece (uppermost drawing) and of the three overlapping trays inside the encasing according to an embodiment of the present invention.
Figure 2 shows a kit comprising a mouthpiece, a control unit, a local light head and a charging dock according to an embodiment of the present invention.
Figure 3 shows in perspective view a portion of a tray comprising rows of light emitting components placed along the rim of the tray in inclined position and some light emitting components placed in an embedded fashion on the biting surface of the tray according to an embodiment of the present invention.
Figure 4 shows a stand-alone implementation of a mouthpiece according to an embodiment of the present invention.
Figure 5 shows a flexible mouthpiece according to an embodiment of the present invention.
Figure 6 shows a top level and a bottom level of a flexible mouthpiece according to an embodiment of the present invention.
Figure 7 shows in perspective view a portion of a top level and bottom level of a flexible mouthpiece according to an embodiment of the present invention.
Figure 8 shows a portion of a body of a mouthpiece according to an embodiment of the present invention.
Figure 9 shows a portion of a body of a mouthpiece according to an embodiment of the present invention.
Figure 10 illustrates a method according to an embodiment of the present disclosure.

It will be appreciated that elements in the figures are illustrated for simplicity and clarity and have not necessarily been drawn to scale. For example, the dimensions of some of the elements in the figures may be exaggerated relative to other elements to help improve understanding of illustrated embodiments of the present disclosure.

### Embodiments

It is to be understood that the embodiments of the invention disclosed are not limited to the particular structures, process steps, or materials disclosed herein, but are extended to equivalents thereof as would be recognized by those ordinarily skilled in the relevant arts. It should also be understood that terminology employed herein is used for the purpose of describing particular embodiments only and is not intended to be limiting.

Reference throughout this specification to one embodiment or an embodiment means that a particular feature, structure, or characteristic described in connection with the embodiment is included in at least one embodiment of the present invention. Thus, appearances of the phrases "in one embodiment" or "in an embodiment" in various places throughout this specification are not necessarily all referring to the same embodiment. Where reference is made to a numerical value using a term such as, for example, about or substantially, the exact numerical value is also disclosed.

As used herein, a plurality of items, structural elements, compositional elements, and/or materials may be presented in a common list for convenience. However, these lists should be construed as though each member of the list is individually identified as a separate and unique member. Thus, no individual member of such list should be construed as a de facto equivalent of any other member of the same list solely based on their presentation in a common group without indications to the contrary. In addition, various embodiments and example of the present invention may be referred to herein along with alternatives for the various components thereof. It is understood that such embodiments, examples, and alternatives are not to be construed as de facto equivalents of one another, but are to be considered as separate and autonomous representations of the present invention.

Furthermore, the described features, structures, or characteristics may be combined in any suitable manner in one or more embodiments. In this description, numerous specific details are provided, such as examples of lengths, widths, shapes, etc., to provide a thorough understanding of embodiments of the invention. One skilled in the relevant art will recognize, however, that the invention can be practiced without one or more of the specific details, or with other methods, components, materials, etc. In other instances, well-known structures, materials, or operations are not shown or described in detail to avoid obscuring aspects of the invention.

When a claim or embodiment recites a member selected from the group consisting of [e.g., elements X, Y, Z], the use of "comprising" elsewhere does not alter the closed meaning of the Markush group. The member is limited to the elements of the group, while the overall claim may include additional features outside the group as allowed by "comprising".

In the present disclosure, the terms "example" or "for example" may be used to represent giving an example, an illustration, or a description. Any embodiment or concept described as an "example" or "for example" should not be understood to necessarily be more preferred or having more advantages than another embodiment.

As used herein, the term "about" refers to a variation of ± 5% or less of the stated value, or such variation as would be understood by one of ordinary skill in the art to be acceptable for the given measurement.

"At least one", "one or more", and "and/or" are open-ended expressions that are both conjunctive and disjunctive in operation. For example, each of the expressions "at least one of A, B, and C", "at least one of A, B, or C", "one or more of A, B, and C", "one or more of A, B, or C" and "A, B, and/or C" means A alone, B alone, C alone, A and B together, A and C together, B and C together, or A, B and C together.

While the below examples are illustrative of the principles of the present invention in one or more particular applications, it will be apparent to those of ordinary skill in the art that numerous modifications in form, usage and details of implementation can be made without the exercise of inventive faculty, and without departing from the principles and concepts of the invention. Accordingly, it is not intended that the invention be limited, except as by the claims set forth below.

The verbs "to comprise" and "to include" are used in this document as open limitations that neither exclude nor require the existence of also un-recited features. The features recited in depending claims are mutually freely combinable unless otherwise explicitly stated. Furthermore, it is to be understood that the use of "a" or "an", that is, a singular form, throughout this document does not exclude a plurality.

As used herein, "intraoral surface" or "intraoral surfaces" include any surface located within the oral cavity of a human, including both hard and soft tissue surfaces. Such surfaces include, without limitation, teeth, gingival tissue, mucosal tissue, the tongue, and the palate, as well as surfaces of dental restorations, prostheses, or any other structures placed within the oral cavity.

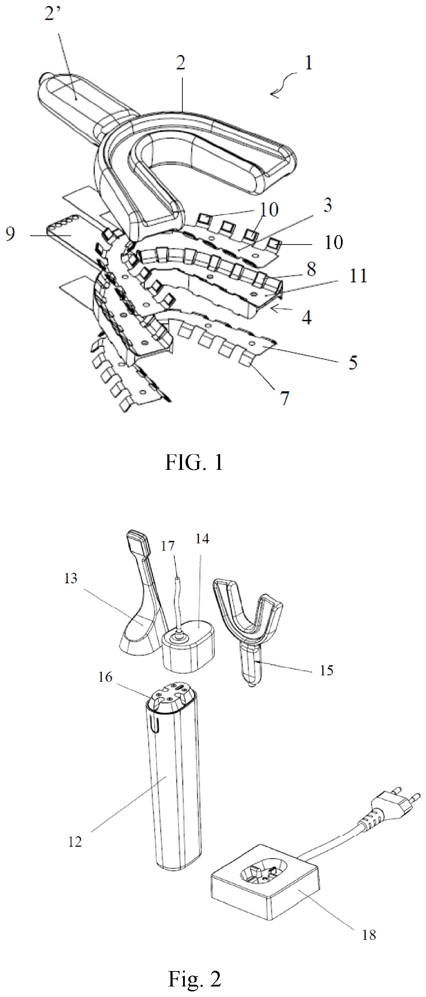

Now referring to the figures, Figure 1 illustrates a mouthpiece 1, the mouthpiece 1 comprising a polymeric cover or encasing 2/2', which can comprise, for example, silicone rubber or thermoplastic, preferably transparent or translucent and which is approved for human use. Further, the mouthpiece 1 comprises a top level 3, a bottom level 5, and plate 4 disposed between the top level 3 and bottom level 5. Reference numerals 3, 4, and 5, and all of their components (light sources 7 (shown from behind), edges 8, cooling tray 9, light sources 10, planar surface 11, may collectively be referred to as a body 12 of the mouthpiece.

In some embodiments, the polymeric cover or encasing 2/2'may comprise or consist or consist essentially of a polymer material approved for oral use, such as silicone rubber or a thermoplastic material or a combination of them. The cover may comprise a single uniform layer or multiple layers of same of different material. The cover may also include optically active components or material which alter the optical properties of the mouthpiece or induce other effects such as antimicrobial effect through reactive oxygen species (ROS) production but not limited to it. Optically active compounds include but are not limited to TiO₂, SiO₂, and doped derivatives thereof, such as derivatives doped with Au, Ag, N, Fe or Cu or combinations thereof.

In one embodiment, the polymeric material forming the encasing contains, mixed therewith or as a coating on the encasing, an antibacterial and/or light diffracting material, such as TiO₂, SiO₂, Ag, metal doped TiO₂, metal doped SiO₂, N (nano)-TiO₂, N (nano)-SiO₂ or silver nanoparticles or combinations thereof.

A thickness of polymeric cover or encasing 2 at a user's biting area of the teeth is over 1 mm, preferably 2-3 mm, optimally up to 10 mm. In some embodiments, the thickness is from 5 to 10 mm, which may be particularly suitable for soft materials.

In some embodiments, the body comprises a heat conducting material having a thermal conductivity of greater than 0.5 W/mK, for example, greater than 1 W/mK. In some embodiments, the thermal conductivity is from 1.1 to 25 W/mK for polymer materials and 100 to 500 W/mK for metal materials.

In some embodiments, the top level 3 and bottom level 5 comprise printed circuit boards (PCBs). In some embodiments, the light sources 10 comprise light emitting diodes (LEDs).

In some embodiments, the light sources are capable of emitting light at an average power density of 30 to 1000 mW/cm², such as 50 to 500 mW/cm², towards an intraoral surface of the user.

In some embodiments, the light sources are configured to emit simultaneously both non-visible treatment light and visible safety light, the visible safety light having an intensity greater than 1.8 cd, preferably greater than 25 cd and even greater than 100 cd. In some embodiments, the intensity is equal to or less than 5000 cd, equal to or less than 3000 cd, or equal to or less than1500 cd.

In some embodiments, the light sources are configured to emit light to achieve a desired treatment intensity and treatment time based on sensory input. In some embodiments, the sensory input is provided an intraoral sensor.

In some embodiments, at least a portion of the light sources are configured to produce light having multiple peak wavelengths.

In some embodiments, at least a portion of the light sources have multiple light emitting surfaces.

In some embodiments, at a position (e.g., bottom) of the U (the arch of the body), there is provided an extended portion which defines cooling surface or tray (9) extending generally in opposite direction to the legs (also referred to herein as distal end portions).

The cooling surface or tray 9 may protrude out from the silicone cover and may incorporate a passive or active cooling function. Active cooling may comprise but is not limited to assisted air cooling, water cooling, oil cooling or Peltier cooling or a combination of them. Passive cooling may comprise but is not limited to air cooling via convection, heat pipe cooling, evaporative cooling or heat storage cooling, radiative cooling or combination of them.

In some embodiments, the edges 8, extending from the inner border or outer border of the planar portions comprise oblique surfaces or consist essentially of such surfaces. At least a part of or all of the edge surfaces are planar. At least a part or all of the edges are tilted so as to form an angle of up to 90 °, in particular about 5 to 85 °, or 10 to 55 °, or 20 to 45 ° with respect to the planar portions of at least one of the top and bottom levels 3, 5 or with respect to the planar surface 11. By placing at least a part of the light sources on at least one of the tilted edges of the body, it is possible to emit light at an angle of up to 90°, in particular 5 to 85°, or 10 to 55 ° against intraoral surfaces (e.g., teeth surfaces and intraoral tissue, such as gum about the teeth).

In some embodiments, the angle of the edges of the body 12 vary in different parts of the mouthpiece, being for example 5 to 30° in a first portion of the body and 25 to 60° in a second portion of the body 12.

In some embodiments, the mouthpiece 1 is provided with one or more temperature sensors configured to measure the temperature of the light sources or their surroundings. In some embodiments, the mouthpiece may include one or more components for continuous monitoring of the temperature and for limiting light source, e.g., LED power, if the temperature rises to a predefined level. This will keep the user safe and allow the light sources to stay in their optimal zone for best thermal efficacy.

Referring again to the figures, Figure 2 illustrates a kit comprising a mouthpiece 15 (which may be a mouthpiece 1 as described above), a control unit, a local light head and a charging dock 18. As shown, the kit further comprises a control electronic circuit 14, 17 which is to be connected to the intraoral mouthpiece 15, in particular the control electronic circuit is to be connected to the intraoral mouthpiece via a male USB port thereof. Further, the kit comprises power source 12, LED head 13, and docking station 11.

The mouthpiece 15 may comprise a built-in electric power reservoir, such as a battery or supercapacitor, for powering the light sources 10 and any other electronic functions that the mouthpiece 15 incorporates. In some embodiments, the power reservoir can be charged wirelessly.

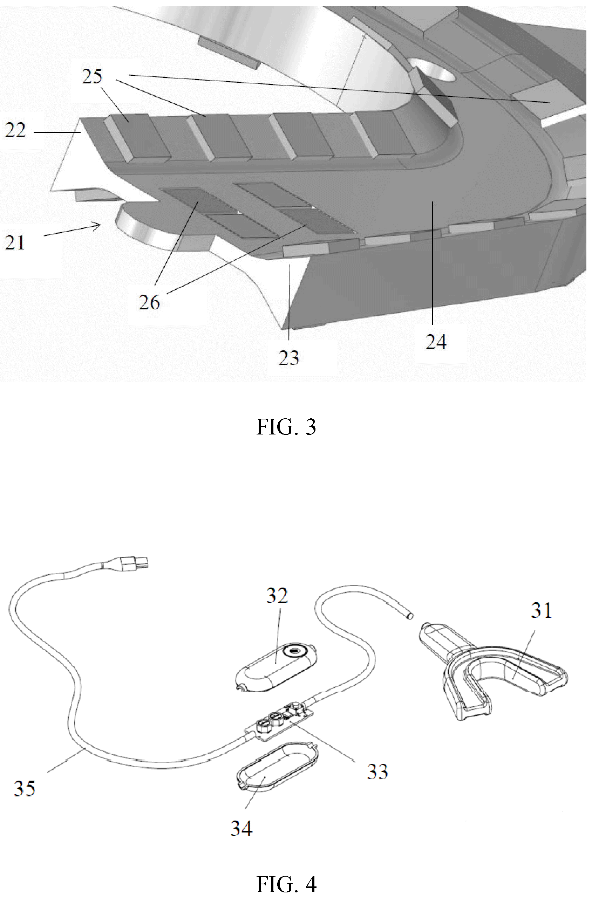

Figure 3 shows a part of a body according to an embodiment of mouthpiece 1, with reference numeral 21 generally referring to the body and numeral 24 which extends from the inner border toward the outer border referring to a planar portion or planar surface of the body 21. Numerals 22 and 23 refer to the edges of the body and numerals 25 and 26 refer to light sources. As shown, a first set of light sources (cf. numerals 25) are located on the inclined edge portions of the body 21. A second set of light sources 26 is located on the planar portions 24 of the body 21. The body 21 may further comprise sets of light sources on the underside of the body 21, in a similar fashion.

Figure 4 shows a stand-alone implementation of a mouthpiece. The stand-alone implementation comprises an intraoral mouthpiece 31 as described herein, and a charger comprising control unit 32, control interface 33, cover 34, and power cable 35.

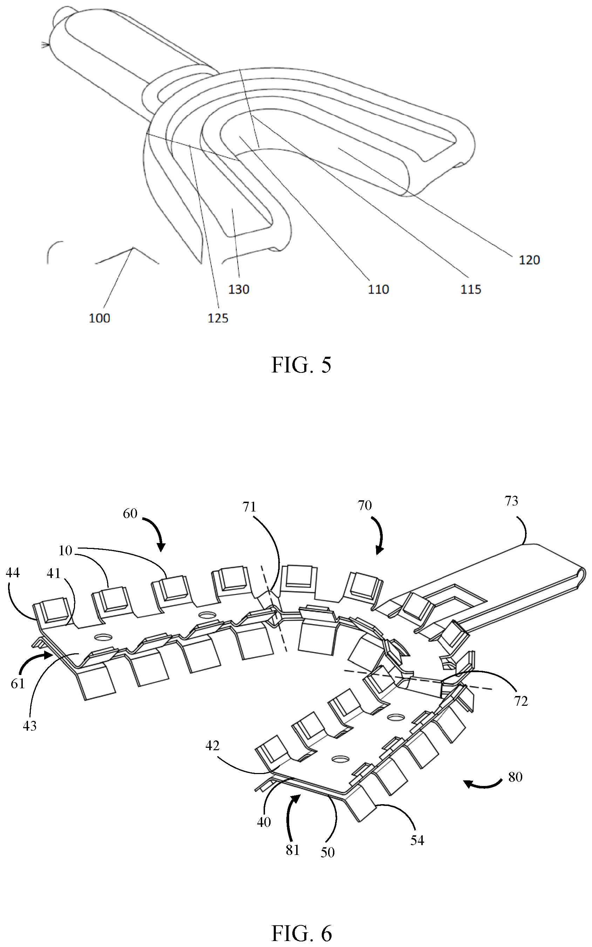

Figure 5 shows a mouthpiece 100 to illustrate the sections of the arch or body. It is understood that the present invention is not limited to the size of the areas shown. The mouthpiece 100 follows the shape of a dental arch and has a central rise portion 110 which is shown between lines 115 and 125. The remainder of the arch consists of a first distal end portion 120 and a second distal end portion 130. It is to be understood that the sections of the arch or the body, i.e., the central rise portion, the first distal end, and the second distal end, describe portions of the mouthpiece inside an encasing when an encasing is provided.

Figure 6 illustrates a top level 40 and a bottom level 50 of a mouthpiece, e.g., mouthpiece 100. In the embodiment shown, the top level 40 and the bottom level 50 are divided into three sections by dashed lines. The three sections are: first distal end portion 60, central rise portion 70, and second distal end portion 80. The first distal end portion comprises an end 61 and the second distal end portion comprises an end 81. End 61 and 81 refer to the back edges of the distal ends positioned furthermost from central rise portion 70. In use, end 61 and end 81 would be positioned towards the back of a user's mouth.

Further, outer border 41 of top level 40 and inner border 42 of top level 40 are illustrated, with planar surface 43 disposed between the inner border 42 and outer border 41. Although not fully visible in the embodiment shown in figure 6, bottom level 50 also comprises an outer border, inner border, and planar surface, similar to the top level. Still further, two light sources 10 have been marked positioned on edges 44 of top level 40. Although not fully visible in the embodiment shown in figure 6, bottom level 50 also comprises a plurality of light sources, positioned on or within edges 54 of bottom level 50.

Additionally, a part of a cooling tray 73 is shown.

In accordance with an aspect of the present invention, the mouthpieces disclosed herein may each comprise one or more flexibility enhancing features to provide a degree of flexibility to the body of the mouthpiece, such as differential flexibility of the central rise portion 70 with respect to distal end portions 60, 80 of the mouthpiece.

In some embodiments, the central rise portion 70 may be formed of a material that has a greater degree of flexibility than a material from which the first distal end portion 60 and the second distal end portion 80 are formed. Any suitable materials may be selected for the central rise portion 70, the first distal end portion 60, and the second distal end portion 80, which provides a differentiation in flexibility. It is understood that the present invention is not so limited to the use of different materials in the central rise portion 70 and the first and/or second distal end portions 60, 80.

In some embodiments, the first distal end portion 60 and/or second end portion 80 are formed from a thermally conductive material having a greater degree of rigidity relative to a material of the central rise portion 70. Exemplary thermally conductive materials for the first distal end portion 60 and the second distal end portion 80 include but are not limited to metals, transparent or translucent thermoplastic or thermosetting materials, silicon-based polymers, polycarbonates, polyacrylates, filled thermoset composites (with added thermal conductivity fillers), liquid crystal polymers, polyimides, and the like. Exemplary materials for the central rise portion 70 include but are not limited to silicone-based elastomers, polyurethane-based systems, and thermoplastic elastomer composites.

In some embodiments, the first distal end portion 60, the second distal end portion 80, and the central rise portion 70 are formed from the same material. In some embodiments, the first distal end portion 60, the second distal end portion 80, and the central rise portion 70 are formed from thermally conductive materials, such as metals, transparent or translucent thermoplastic or thermosetting materials, silicon-based polymers, polycarbonates, polyacrylates, filled thermoset composites (with added thermal conductivity fillers), liquid crystal polymers, polyimides, and the like.

In some embodiments, the central rise portion 70 may be made more flexible than the first distal end portion 60 and the second distal end portion 80 by any suitable method or structure, including by providing grooves in the central rise portion 70 and/or providing the central rise portion 70 with a lesser thickness relative to the first distal end portion 60 and/or second distal end portion 80.

In some embodiments, the central rise portion 70 provides a degree of flexibility effective to allow an end 61 of the first distal end portion 60 and/or an end 81 of the second distal end portion 80 to flex at least 0.5 mm, 1 mm, 2 mm, 3 mm, 4 mm, 5 mm or 10 mm relative to a resting position of end 61 of the first distal end portion 60 and/or of the end 81 of the second distal end portion 80. In some embodiments, the central rise portion 70 provides a degree of flexibility effective to allow the end 61 of the first distal end portion 60 and/or the end 81 of the second distal end portion 80 to flex (inwards or outwards from the arch / U-shape) from 0.5 to 10 mm, such as from 1 to 7 mm, or from 2 to 5 mm, relative to a resting position of the end 61 of the first distal end portion 60 and/or of the end 81 of the second distal end portion 80.

It is appreciated that the size or area of the central rise portion 70 may be selected to provide the desired degree of flexibility to the central rise portion 70. In some embodiments, the central rise portion 70 has a smaller area than the area of each of the first distal end portion 60 and the second distal end portion 80, such as 1.1x, 2x, 5x, 10x, 20x less than the area of each of the first distal end portion 60 and the second distal end portion 80.

Figure 6 further illustrates another flexibility-enhancing feature in accordance with some embodiments of the present disclosure. In the embodiment of figure 6, the flexibility-enhancing feature comprises a first corrugated section 71 extending from the central rise portion 70 towards or to the first distal end portion 60, and a second corrugated section 72 extending from the central rise portion towards 70 or to the second distal end portion 80.

In some embodiments, the corrugated sections 71, 72 may comprise a thinner material than the adjacent materials of the distal end portions 60, 80 and/or the central rise portion 70. The corrugated sections 71, 72 may comprise a thin sheet of material in the form of a wave, which provides flexibility by functioning as a flexible joint which allows bending.

In some embodiments, the corrugated sections 71, 72 may comprise a wavy sheet having a thickness of less than 2 mm, or less than 1 mm, or less than 0.5 mm, or less than 0.3 mm, less than 0.2 mm, or less than 0.1 mm. In some embodiments, the sheet is formed of metal, but may be any other suitable material which is safe for use in a user's mouth.

In some embodiments, the corrugated sections 71, 72 may comprise a wavy metal sheet having a length of at least 2 mm, or at least 4 mm, or at least 6mm, or at least 8 mm, or at least 10 mm.

The corrugated sections 71, 72 may have a length to thickness ratio of at least 2:1, or at least 3:1, or at least 5:1, or at least 8:1, or at least 10:1.

In some embodiments, the corrugated sections 71, 72 are effective to allow an end of the first distal end portion 60 and/or an end of the second distal end portion 70 to flex at least 0.5 mm, 1 mm, 2 mm, 3 mm, 4 mm, 5 mm or 10 mm relative to a resting position of the end of the first distal end portion 60 and/or of the end of the second distal end portion 70. In some embodiments, the corrugated sections 71, 72 are effective to allow the end 61 of the first distal end portion 60 and/or the end 81 of the second distal end portion 80 to flex (inwards or outwards from the arch / U-shape) from 0.5 to 10 mm, such as from 1 to 7 mm, or from 2 to 5 mm, relative to a resting position of the end 61 of the first distal end portion 60 and/or of the end 81 of the second distal end portion 80.

In some embodiments, the top level 40 and the bottom level 50 may comprise corrugated sections 71, 72 vertically opposite to each other.

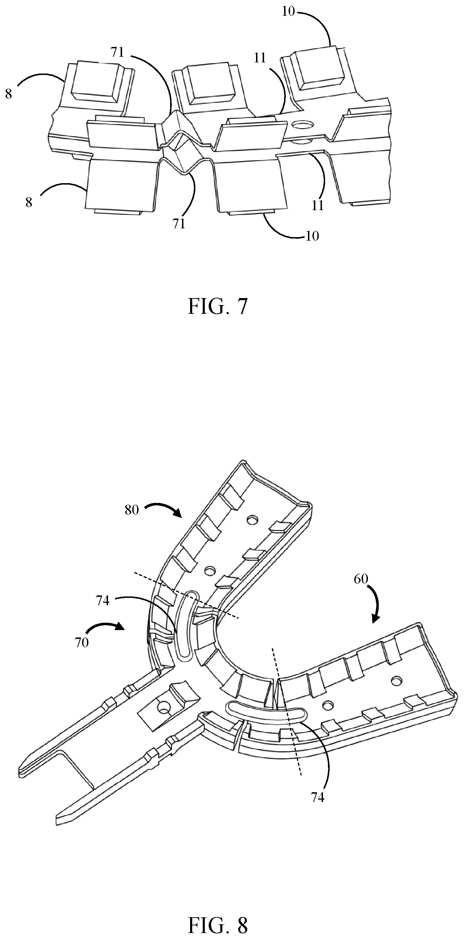

Figure 7 provides a sectional view of the corrugated section 71 of the top level 40 and the bottom level 50. Additionally, edges 8, light sources 10, and planar surfaces 11 of both the top level 40 and bottom level 50 are marked.

Figure 8 illustrates a portion of a body 12 comprising a flexibility-enhancing feature according to another embodiment. In some embodiments, the flexibility-enhancing feature comprises a section of a thermally softening material 74 extending from the central rise portion 70 towards and into the first distal end portion 60 and a section of a thermally softening material 74 extending from the central rise portion 70 towards and into the second distal end portion 80. The thermally softening material 74 is configured to soften and increase flexibility upon heating. The thermally softening material 74 may extend vertically through the top level 40, bottom level 50, and any middle layer such as a tray, or may be positioned on top of the top level 40 and/or on top of the bottom level 50.

The thermally softening material may cover at least 2%, 5%, 10%, 20%, 30%, 40%, or 50% of the total area of the central rise portion 70 of each level 40, 50. Alternatively, the thermally softening material may form at least 2%, 5%, 10%, 20%, 30%, 40%, or 50% of the total volume of the central rise portion 70 of the intraoral mouthpiece.

The thermally softening material may be softened by heating the thermally softening material to a temperature of at least 40 °C, 50 °C, 60 °C, 70 °C, 80 °C, 90 °C, or 100 °C.

In some embodiments, the central rise portion 70 comprises one section of a thermally softening material. In some embodiments, the central rise portion 70 comprises at least two, at least three, or at least four sections of a thermally softening material.

In some embodiments, the thermally softening material is effective, while being heated, to allow an end 61 of the first distal end portion 60 and/or an end 81 of the second distal end portion 80 to flex at least 0.5 mm, 1 mm, 2 mm, 3 mm, 4 mm, 5 mm or 10 mm relative to a resting position of the end 61 of the first distal end portion 60 and/or of the end 81 of the second distal end portion 80. In some embodiments, the thermally softening material is effective to allow the end 61 of the first distal end portion 60 and/or the end 81 of the second distal end portion 80 to flex (inwards or outwards from the arch / U-shape) from 0.5 to 10 mm, such as from 1 to 7 mm, or from 2 to 5 mm, relative to a resting position of the end 61 of the first distal end portion 60 and/or of the end 81 of the second distal end portion 80.

Once the thermally softening material begins to cool down, the distal end portions begin to retain a resting position. Once fully cooled down, the distal end portions fully retain a resting position.

In some embodiments, the thermally softening material is selected from the group consisting of thermoplastic polymers and other heat-moldable polymers.

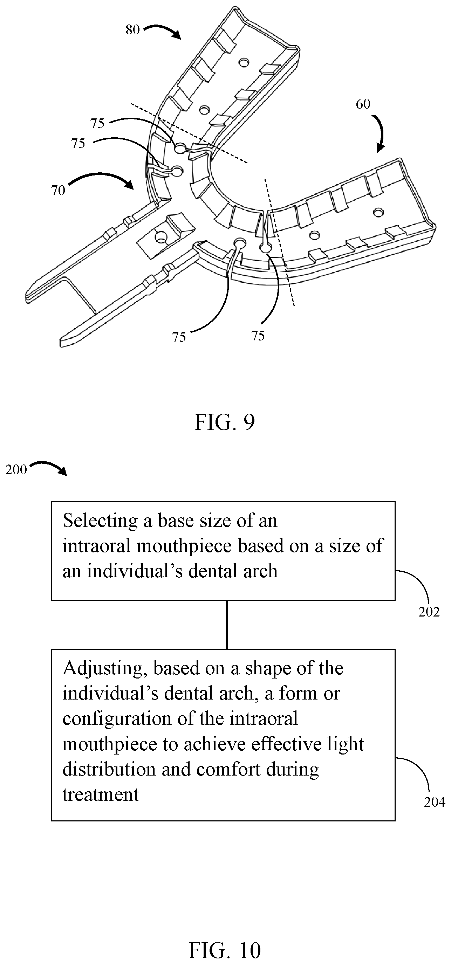

Figure 9 illustrates a portion of a body of a mouthpiece comprising a flexibility-enhancing feature according to another embodiment. Here, the flexibility-enhancing feature comprises perforations 75 positioned on both sides of the central rise portion 70. The perforations are configured to allow permanent mechanical deformation of the body, for example by widening or narrowing the arch (or U-shape) by pulling or pushing or otherwise moving the ends 61, 81 of the distal end portions 60, 80. The mechanical deformation may also be performed by reforming the intraoral mouthpiece over an adjustable forming jig.

A single perforation may extend from the inner border or the outer border of the top level 40 or the bottom level 50 (or of any tray, for example tray 4 illustrated in figure 1) towards an opposite border. A single perforation may extend at least 3 mm, or at least 5 mm, or at least 10 mm, from the inner border 42 towards the outer border 41 or from the outer border towards the inner border.

A single perforation may have width of at least 0.1 mm, or at least 0.3 mm, or at least 0.5 mm, or at least 1 mm, or at least 2 mm, or at least 3 mm in a narrowmost part of the perforation. In some embodiments, a single perforation may have a width of from 0.1 mm to 3 mm, or from 0.5 mm to 2 mm, in a narrowmost part of the perforation.

A perforation may extend vertically through a top level, a tray, and a bottom level, so that a vertical window is formed through the body, as shown in figure 9, for example.

In some embodiments, the intraoral mouthpiece may comprise a plurality of flexibility-enhancing features in combination. For example, the intraoral mouthpiece may comprise one or more corrugated sections, one or more perforations, and one or more thermally softening materials or sections of a thermally softening material disposed on or within the body.

Figure 10 illustrates a method 200 for adjusting the fit of an intraoral mouthpiece for an individual in accordance with some embodiments of the present disclosure. The first step of method 200 is step 202 of selecting a base size of an intraoral mouthpiece (an intraoral mouthpiece as described herein) based on a size of the individual's dental arch. There may be two, three, four, or five base sizes of a mouthpiece. The selection may be based on a determination of a size and shape of an individual's dental arch.

Method 200 further comprises step 204 of adjusting, based on a size and shape of the individual's dental arch, a form or configuration of the intraoral mouthpiece to achieve desired light distribution and comfort during treatment. The adjusting comprises reforming the intraoral mouthpiece over an adjustable forming jig.

During reforming, the end of the first distal end portion and/or the end of the second distal end portion may be pushed or pulled or otherwise moved inwards or outwards of the arch/U-shape. This movement of the ends is enabled by one or more of the flexibility-enhancing features as described herein.

The adjusting may further comprise heating the intraoral mouthpiece prior to the reforming, and cooling the intraoral mouthpiece, allowing the intraoral mouthpiece to set in an optimal position for the individual.

Further embodiments of the present invention are described below.

In some embodiments, it is an object of the invention to solve at least some of the above-mentioned shortcomings and to provide a novel mouthpiece for intraoral antibacterial treatment.

In some embodiments, It is an object of the present invention to provide an oral treatment device comprising an intraoral mouthpiece.

In some embodiments, it is an object of the present invention to provide a kit comprising an oral treatment device.

In some embodiments, it is an object of the present invention to provide a method of delivering light within the intraoral mouthpiece intended for photodynamic treatment of teeth and intraoral tissue.

In some embodiments, it is an object of the present invention to provide a method of treatment of intraoral tissues of a user.

In some embodiments, it is an object of the present invention to provide an intraoral mouthpiece that provides a degree of flexibility such that a given mouthpiece will fit a greater number of users relative to known mouthpieces that deliver photodynamic treatment.

In some embodiments, the various sections of the mouthpiece may be formed by any suitable method in the art. In some embodiments, the sections may be formed by overmoulding rigid portions of the mouthpiece having light sources on or therein with an elastomeric material, wherein the rigid portions define one or more gaps. In this way, the elastomeric material fills the gap portion and defines the more flexible central rise portion.

In some embodiments, any known intraoral mouthpiece or mouthpiece to be developed may incorporate the more flexible central rise portion and first and second distal end portions as described herein. Exemplary mouthpieces include but are not limited to those disclosed in the present application and in US patent application nos. 17/288,231, 17/767,950 and US Patent No. 12/161,179, the entirety of each of which are hereby incorporated by reference.

In some embodiments, the mouthpiece may comprise a body of a heat conducting material. The body generally exhibits an upper surface and an opposite lower surface, which surfaces typically comprise an essentially planar portion between two opposite edges, at least one lingual edge and at least one buccal or labial edge. The upper and lower surfaces further contain a plurality of light sources attached to the body and adapted to deliver light to teeth surfaces or oral tissue. As used herein, the term "oral tissue", when used in this description, refers to all oral tissue except for the teeth, such as the cheeks, tongue, palate (both hard and soft), tonsils, cheek lining, floor of the mouth, salivary glands, and mucous membranes.

In some embodiments, opposite edges are provided with surfaces, at least a part of which are planar and tilted so as to form in cross-section of the body an angle of up to 90° with respect to the planar portions of the upper and lower surfaces. At least a part of the light sources are preferably positioned on at least one of the tilted buccal, labial or lingual edges of the body. The body and the light sources are embedded in an encasing formed by a transparent polymeric material. Such material will protect the body while still allowing for light to pass through the encasing material to the teeth and tissues while the mouthpiece is placed in use position in the mouth of an individual.

In one embodiment, in the device the tilted edges form an angle of 5 to 75°, for example 10 to 65°, with regard to the planar portions of the upper and lower surfaces.

In one embodiment, the tilted edges form an angle of 10 to 55° , in particular 30 to 50°, with regard to the planar portions of the upper and lower surfaces.

In some embodiments, the kit comprises an intraoral mouthpiece, preferably of the kind explained in the foregoing, with a body made of a heat conducting material combined with a control electronic circuit connected to the body, in particular connected to the body via a male USB port thereof.

In some embodiments, the kit may also comprise an intraoral mouthpiece together with an intraoral agent, in particular an active agent selected from the group of benefit agents, including antioxidants, photosensitizers and potentiating agents and combinations thereof.

In some embodiments, the present mouthpiece allows for the delivery of light for photodynamic treatment of teeth and intraoral tissue. In use, at least a part of the light sources are positioned on at least one of said tilted buccal, labial or lingual edges of the body, and the light sources are supplied with electric power for delivering light towards the user's teeth and intraoral tissue.

In some embodiments, the mouthpiece is placed in the user's mouth such that the upper and lower surfaces engage at least the chewing surface of the user's teeth; the light sources are supplied with electric power for generating light; and the light is directed towards the user's teeth at an angle with respect to the plane of the chewing surface of the teeth to achieve photodynamic oral treatment of the teeth and oral issue.

In one aspect, considerable advantages are obtained with the present technology of the present technology will contribute to improving efficiency of treatment and mitigate heat build-up, which typically and interrelated aims. The present invention also provides for improved user safety and manufacturability.

In some embodiments, the light sources are LEDs (light emitting diodes) which will give high intensity of light at a preselected wavelength with low heat emission.

In some embodiments, the mouthpiece is capable of distributing light evenly to dental and periodontal locations, when a light source, such as a LED light source, is located at the facial or lingual surface of the teeth. By placing the light sources, in particular the LEDs facially or lingually, i.e. in the area between the lips or cheeks and the tooth, or between the teeth and the tongue, any thermal loss of the light source(s) will be absorbed by the surrounding tissues during the light exposure. Furthermore, a placing of the light sources, such as LEDs, away from the occlusal surfaces of the teeth, reduces the risk of breaking the LED part by biting. Optical attributes of the modular elements in the mouthpiece enable an even light distribution on all surfaces of the teeth.

In some embodiments, the LED elements may have an over 35% thermal efficacy.

In some embodiments, the present disclosure relates to mouthpieces and methods for antibacterial treatment of intraoral surfaces.

In some embodiments, the mouthpiece comprises a body made of heat conducting material, the body comprising tilted facial and lingual outer surfaces and facial and lingual inner surfaces adapted to face facial and lingual surfaces of teeth, respectively. A light source is attached to the body's tilted surface and adapted to deliver light to surfaces of teeth, the light source being positioned on at least one of said outer surfaces and adapted to deliver said light via said inner surfaces to both the facial and lingual surfaces of the teeth.

In one embodiment, an oral treatment device comprises an intraoral mouthpiece which comprises a body made of a heat conducting material which exhibits an upper surface and an opposite lower surface. The surfaces are configured such that there is, preferably on both opposite sides of the generally planar body, surfaces comprising a planar or essentially planar portion which is located between edges, on at least the outer side, preferably on both opposite sides.

Thus, in one embodiment, there is a lingual edge and a buccal and/or labial edge. The planar portion or preferably planar portions are configured to be placed against the surfaces of the user's teeth when the mouthpiece is placed in the mouth of the user between the teeth. Thus, the planar surfaces of the body will abut with the biting surfaces of the teeth.

In some embodiments, the body further comprises at least one, but preferably a plurality of light sources attached to the body and adapted to deliver light to teeth surfaces. Generally, there are about 1 to 20 light sources on each lingual side and 1 to 10 light sources labially. By providing the body with a plurality of light sources, it is possible to ensure an even emission of light against the teeth and gum areas or other intraoral areas.

Furthermore, in some embodiments, the edges, arranged on either or both sides of the planar portions comprise oblique surfaces or consist essentially of such surfaces. At least a part of or all of the edge surfaces are planar. The surfaces are further at least partially and tilted so as to form an angle of up to 90°, in particular about 5 to 85° with respect to the planar portions of at least one of the upper and lower surfaces. By placing at least a part of the light sources on at least one of the tilted buccal, labial or lingual edges of the body it is possible to emit light at an angle of up to 90°, in particular 5 to 85° against the teeth surfaces and the intraoral tissue, such as gum about the teeth.

It should be noted that the angle of the edges of the body can vary in different parts of the mouthpiece, being for example 5 to 30° in a first portion of the body and 25 to 60° in a second portion of the body.

In some embodiments, the mouthpiece preferably comprises an encasing for the body and the light sources. In particular, the body and the light sources are embedded in an encasing formed by a transparent polymeric material.

In one embodiment, the shape of the body onto which the light sources are assembled allows provision of LEDs on the base surface of the body at an angle of 90° or less, but preferably 5° or more, to direct light towards the teeth. The adjustment of the treatment and light intensity can be done by directing the LEDs towards teeth or towards gum or other intraoral tissue by adjusting the angle in the base surface of the body.

In one embodiment, the tilted edges form an angle of 10° or more, in particular more than 20°, ° or at least 25°, preferably at least 30 °, with regard to the planar portions of the upper and lower surfaces. Thus, in one embodiment, the tilted edges form an angle of more than 20° or at least 30° and up to 85°.

In one embodiment, the tilted edges form an angle of 10 to 55°, in particular 30 to 50°, with regard to the planar portions of the upper and lower surfaces.

The present technology also provides, in an embodiment, a method of delivering light within an intraoral mouthpiece intended for photodynamic treatment of teeth and intraoral tissue.

In one embodiment, the method comprises the steps of providing a body made of a heat conducting material, the body further having an upper surface and an opposite lower surface, said surfaces comprising an essentially planar portion between lingual edges and buccal or labial edges, respectively, and configured to be placed against teeth surfaces.

In one embodiment, at least a part of which are planar and tilted so as to form in cross-section of the body an angle of 5 to 85° with respect to the planar portions of the upper and lower surfaces.

In one embodiment, there are positioned on at least a part of the edges of the body light sources to the body which are adapted to deliver light to teeth surfaces and intraoral tissue, and electric power is supplied to the light sources.

In one embodiment of a method of treatment of a user's teeth and intraoral tissue, a mouthpiece as disclosed herein is placed in the user's mouth such that the upper and lower surfaces engage at least the chewing surface of the user's teeth; the light sources are supplied with electric power for generating light; and the light is directed towards the user's teeth at an angle, in particular an oblique angle, with respect to the plane of the chewing surface of the teeth to achieve photodynamic oral treatment of the teeth and intraoral tissue.

In some embodiments, the mouthpiece may comprise one or more of the following features shown in Figure 1:
- A polymeric cover or encasing 2, which can comprise silicone rubber or thermoplastic, preferably transparent or translucent and approved for human use;
- Sheet or layers 3, 5, preferably flexible and each comprising, in particular, a printed circuit board;
- Light emitting components (light sources) 10, for example comprising light emitting diodes (abbreviated LEDs); and
- A body in the form of a sheet or plate 4 which generally is U-shaped (in planar direction) and preferably rigid.

In one embodiment, in the present context, the term "rigid" denotes that the body (i.e. sheet or plate) 4 is not deformed during normal use of the mouthpiece - for example it is not essentially bent in either longitudinal or transversal direction by the biting force exerted by the user of the mouthpiece.

In one embodiment, the sheet or plate 4 comprises in particular a thermally conducting material. In one embodiment, the body comprises a generally U-shaped arc (3-5) so as to be able to follow a dental arc.

In one embodiment, structure 4 may be referred to in the following as a "tray" or a "heat dissipating" (or "hear conducting") tray. The tray typically has an extended portion, in particular at the front end (in use position), which forms a cooling tray or surface 9.

Thus, in one embodiment, at the far end (i.e. bottom) of the U, on the opposite side to the legs of the U, extending generally in opposite direction to the legs, there is preferably an extended portion which in particular functions as a cooling surface or tray.

Similarly, Figure 3 shows a part of a tray according to an embodiment, with reference numeral 21 generally referring to the tray and numeral 24 referring to the planar portion of the tray 21. Numerals 22 and 23 refer to the inclined edge portions of the tray and numerals 25 and 26 refer to light sources. As will appear, a first set of which (cf. numerals 25) are located on the inclined edge portions of the tray 21. A second set 26 is located on the planar portions 24 of the tray 21.

In one embodiment, the tray typically comprises a body of metal or of a heat conducting polymer or a combination thereof. The preferred heat conduction (also known as "thermal conductivity") for the material of the body (in the following also "base") is greater the 0.5 W/mK. For metal bases it is typically between 1 and 1000 W/mK, preferably 5 to 400 W/mK, in particular 50 to 250 W/mK. The preferred heat conduction for a polymer base is between 0.5 to 100 W/mK, preferably 1 to 20 W/mK, such as 5 to 15 W/mK. The afore-going values stand for "thermal conductivity in plane" and can be determined by the ASTM E1461.

In one embodiment, the tray 4; 21 will allow for the assembly of light sources into the mouth piece such that they are capable of directing light at an angle, in particular an oblique angle towards teeth. To that end, the tray 4; 21 comprises planar area portions 11; 24 defined between opposite edge portions 8; 22, 23 which are tilted or include with respect to the planar portions. As a result, as will appear from Figures 1 and 3, the tray at least partially exhibits a time glass shaped cross-section. As also will appear, the edges are tilted so as to form a slope towards the planar portions of the upper and lower surfaces.

In one embodiment, the body comprises light reflecting, light guiding or both light reflecting and light guiding elements positioned at the edges of the upper and lower surfaces of the body, said elements being capable of reflecting or guiding, or both, light emanating from said light sources.

In one embodiment, the light emitting components can be embedded in the rigid tray to increase the light to biting surface and protect the component from mechanical stress. One possible option is to use two sided flexible circuit boards and have asymmetrical holes in the tray to allow light to travel to opposite side of the mouthpiece.

In one embodiment, the adjustment of the treatment and light intensity can be done by adjusting the position of the light emitting surfaces of the light sources with respect to the teeth. Thus, the light sources can be configured to emit light towards the teeth or gum or both. It is also possible to adjust the positions of the supporting edges 6, 7; 22, 23 of the tray and/or to adjust the thickness of the polymer cover to adjust light distribution.

In one embodiment, the light sources provide light which is emitted against the teeth or gum or both at an acute or oblique angle. In one embodiment, the angle of the light emitting surfaces of the light sources varies between 0 and 90 degrees, preferably light is directed against the teeth or gum or both at an angle of about 5 and 85 degrees, for example between 30 and 60 degrees, and in particular 35 to 50 degrees. The angle refers to the direction of the incident light waves having the greatest energy of the light emitted by the light sources.

In one embodiment, when using LEDs or similar light sources which have an at least essentially light emitting surface, it is preferred that that surface be configured to emit light at an oblique angle, said angle preferably being about 5 to about 85°, with respect to a plane defined by the biting surfaces of the teeth. To that end, the light sources can be mounted on edge portions of the tray which stand at an oblique angle on either or both sides of a planar surface of the tray. In particular, such a planar surface is preferably configured to abut with the biting surfaces of the teeth when the mouthpiece is being used.

As shown in Figure 3, the tray may further have light sources, such as LEDs 23, embedded into the biting surfaces to increase the light intensity directed to biting surface of the teeth. The light sources can be configured as flexible printed circuit boards. Typically, the tray 4; 21 supports two strings of LEDs on each side of the tray as shown in Figure 1. The light sources may comprise a multi-LED strip positioned at and preferably arranged to follow the shape of the buccal, labial or lingual edges of the body. The light sources can be are positioned on the buccal or labial side, or both, of the body. They can also be positioned on the lingual outer surface of the body.

In one embodiment, the light sources are adapted to deliver light to the tongue through the lingual outer surface of the body.

In one embodiment, the light sources, such as LEDs, used in the mouthpiece are capable of emitting light having an average power density of 30 to 1000 mW/cm², in particular 50 to 500 mW/cm², towards the teeth.

The light sources can be connected to any kind of circuit board including, but not limited to, flexible PCB, copper strips, metal PCB, semi flexible PCB and combination of them.

The thermal junction between the tray and the light emitting element can be increased by thermal conductive material such as thermal paste or thermal conductive glue (not-shown in the drawings).

In one embodiment, the tray 4; 21 dissipates heat from the hotspots around the mouth where the heat is dissipated in controlled way to oral surfaces of user's mouth. Allowing good light power and solving the issue of light emitting component overheating or formation of local hotspots.

As shown in the embodiment of Figure 1, the part 9 of the tray 4 external to mouth can protrude out from the silicone cover and it can incorporate a passive or active cooling function 2'. The tray 4 can further have grooves or pipes to allow cooling liquid or air to travel inside the tray. Active cooling can comprise but is not limited to assisted air cooling, water cooling, oil cooling or Peltier cooling or combination of them. Passive cooling can comprise but not limited to air cooling via convection, heat pipe cooling, evaporative cooling or heat storage cooling, radiative cooling or combination of them.

Based on the above, in one embodiment, the heat conducting body 3-5, 9 contains an extended portion which forms a cooling tray or surface 9, which is capable of conducting heat from the body 3-5 inside an encasing 2 to the outside of the encasing. Typically, the extended portion is placed at the front end of the tray (when the mouth piece is placed in position between the teeth).

In one embodiment, the tray is connected to an active cooling unit for cooling the body inside the encasing, said active cooling being connected to a temperature sensor to allow for adjustment of cooling of the body depending on the temperature of the mouthpiece.

In one embodiment, with LEDs at biting surface 23 the thickness of the cover is over 0.1 mm preferably over 1 mm and up to 10 mm at the most. In one embodiment, the body embedded in the encasing is capable of enduring a temperature of up to 100 °C during the operation of the device.

In one embodiment, the invented mouthpiece may be used as a kit comprising an interchangeable mouthpiece and a local light illuminator head and a control unit and a charging dock to be used together with an active substance.

In one embodiment, the mouthpiece should be used as a kit comprising an interchangeable mouthpiece and a local light illuminator head and a control unit and a charging dock to be used together with an active substance.

Further embodiments relate to mouthpieces comprising a body made of heat conducting material, the body comprising tilted facial and lingual outer surfaces and facial and lingual inner surfaces adapted to face facial and lingual surfaces of teeth, respectively. A light source is attached to the body's tilted surface and adapted to deliver light to surfaces of teeth, the light source being positioned on at least one of said outer surfaces and adapted to deliver said light via said inner surfaces to both the facial and lingual surfaces of the teeth.

In one embodiment, the encasing includes bristles, shapes, rods capable of brushing teeth and electric motor capable of moving or vibrating bristles, shapes, and rods and combinations thereof.

Referring to Figure 3, it can be noted that the light sources 10 are connectable to a power source 12, such as a built-in electric power reservoir, for powering the light sources.

In one embodiment, the mouth piece of the oral treatment device is connectable to a USB-C port.

In one embodiment, the kit comprises intraoral agent, in particular an active agent selected from the group of benefit agents, including antioxidants, photosensitizers and potentiating agents and combinations thereof. Thus, for example, the intraoral agent can be selected from the group of antioxidant molecules, such as vitamin E or an analog or precursor thereof, pigments, and combinations thereof.

Specific examples of active agents, in particular intraoral agents comprise photosensitizers selected from the group of Hypericin, curcumin, phenalenone derivatives, Cercosporin, psoralen, xanthotoxin, Angelicin, alpha-Terthienyl, Phenylthepatriyne, THC, Cannabidiol (CBD). Synthetic photosensitizers include the following: RB (Rose Bengal), MB, Porphyrin derivatives, Curcumin derivatives, Methylene Blue, Indocynine Green, Erythosine, Phenalenone derivatives, Fullerene derivatives, Xanthene derivates, optionally together with a pigment.

In one embodiment, the intraoral agent is selected from indocyanine green in combination with titanium dioxide, optionally further combined with at least one antioxidant, such as E-vitamin or an analogue or precursor thereof. In one embodiment, the mouthpiece comprising structure(s) for mechanically vibrating the body while positioned intraorally.

Power can be switched on for example automatically when disconnected from the charger or manually when the user bites the mouthpiece, as detected by a suitable sensor.

In one embodiment, the power storage is implemented using one or more supercapacitors which can drive the device at least for 1 minute, preferably at least for 2 minutes, for example 5 minutes and up to 120 minutes, in particular at least 15 minutes and up to 30 minutes. There may for example be one supercapacitor per one or a group of LEDs.

The device can also have one or more sensors. This allows for providing a measurement and/or feedback functionality to the treatment process. In some embodiments, at least one sensor is provided for measurement of light absorption of LED components from LED current. In alternative or supplementary embodiments, there may be provided at least one photosensitive element, such as a diode, capable of measuring light absorption. Light absorption information can be linked to amount of biofilm on top of teeth and to early onset of caries.

In some embodiments, the mouthpiece is provided with a sensor for measure change in absorbed light of the emitted wavelength or wherein the component associated with the sensor output emits light of a first wavelength, and the sensor input detects light of a second wavelength different than the first wavelength.

In some embodiments, the mouthpiece is provided with one or more temperature sensors adapted so measure the temperature of the light sources or their surroundings. There may also be means for continuous monitoring of the temperature and for limiting LED power if the temperature rises to a predefined level. This will keep the user safe and allow LEDs to stay in their optimal zone for best thermal efficacy.

In some embodiments, the mouthpiece surface is at least partially coated with small layer of titanium dioxide that will produce reactive oxygen as 405 nm light is emitted from the structure. Titanium dioxide can be used as the diffusive or diffracting material in the mouthpiece and it also has a therapeutic bacteria-killing function as such.

In some embodiments, the It is also possible to make light weight photodynamic treatment device for low effect local use by using only one or two modular elements with a limited number of electronic components. Charging of mouthpiece is done wirelessly to a super capacitor. The device lights up when lifted from charger.

In some embodiments, the mouthpiece is provided with a self-antibacterial function by having an antibacterial layer thereon and irradiating the antibacterial layer, for example periodically when in standby-mode, e.g. in a charger. For example, the mouthpiece may provide 405 nm light to titanium dioxide particles contained in the mouthpiece.

In one embodiment, the treatment is started by applying the active ingredient product to mouth and then placing the mouthpiece into the mouth and connecting the mouthpiece with an external control unit by a cord or wirelessly. The control unit can be a mobile device which is easy to carry during the treatment. After completed treatment, the user may take a probiotic product in form of gum, tablet, paste or liquid, for example.

In one embodiment, the mouthpiece comprises at least one sensor, such as an optical sensor, for measuring the response of intraoral tissue or intraoral agent to light emitted thereto, such as photobleaching of active ingredient or the amount of active ingredient in the treatment area.

In some embodiments, the present photodynamic therapy mouthpiece has one or more safety features that prevent user to harm him-/herself with high intensity invisible light. In one embodiment, a safety feature is based on regulating the brightness of the light source(s) for light components outside of 390-700 nm wavelengths based on a sensor input of the device or include additional light source of emitting light in the visible 390-700 nm spectrum to generate the natural and active aversion response of the eye to bright light. The device may additionally be configured to turn from low power to high power state once detecting target wavelength from treatment area that is inherent to a target molecule. In some embodiments, the device utilizes 780-815 nm light as primary active light and bright white light as eye-protecting light.

In some embodiments, the device is configured such that it switches from low power to high power treatment radiation only after detecting fluorescence radiation from its target area by use of a sensor, and thus protection of the eyes and other tissues from unnecessary exposure to radiation is achieved.

In one embodiment, the light sources are configured to emit light within at least one non-visible wavelength band, such as within a band of 780-820 nm.

In one embodiment, the mouthpiece comprises an intraoral sensor and means for regulating the output power of the light sources on at least one wavelength, in particular at least one non-visible wavelength band, based on input from the intraoral sensor.

In one embodiment, the light sources are configured to deliver light, in particular non-coherent light, at a first wavelength from 400 to 430 nm (corresponding to photons of about 3.1 eV to 2.9 eV), preferably at a dosage of 1 to 120 J/cm², and in particular with a power density of from about 10 to about 2500 mW/cm² for a period of time from 0.5 s to 120 min, and at a second wavelength from 780 to 830 nm (corresponding to photons of about 1.59 eV to 1.49 eV) , preferably at a dosage of 1 to 120 J/cm², and in particular with a power density of from about 10 to about 2500 mW/cm² for a period of time from 0.5 s to 120 min.

### Industrial applicability

At least some embodiments of the present invention find industrial application in oral health applications, more specifically in treatment of intraoral surfaces.

## Claims

1. An intraoral mouthpiece comprising:
a body comprising a heat conducting material, the body defining an arch and having a top level, a bottom level opposite the top level, each level having an inner border and an outer border opposite the inner border, and a planar surface disposed between the inner and outer border of each level; and
a plurality of light sources located on or within the body and configured to deliver light to intraoral surfaces of a user when worn,
wherein the body further comprises a central rise portion disposed between a first distal end portion and a second distal end portion and at least one flexibility-enhancing feature to provide flexibility to at least a portion of the body.

2. The mouthpiece of claim 1, wherein the at least one flexibility-enhancing feature comprises the central rise portion being formed from a material having a greater degree of flexibility than a material from which the first distal end portion and/or the second distal end portion are formed.

3. The mouthpiece of claim 1 or 2, wherein the first distal end portion and/or the second distal end portion comprises one or more materials selected from the group consisting of metals, transparent or translucent thermoplastic or thermosetting materials, silicon-based polymers, polycarbonates, polyacrylates, filled thermoset composites, liquid crystal polymers, polyimides, and the like.

4. The mouthpiece of any one of the previous claims, wherein the central rise portion comprises a material selected from the group consisting of silicone-based elastomers, polyurethane, and thermoplastic elastomer composites.

5. The mouthpiece of any one of the previous claims, wherein the at least one flexibility-enhancing feature comprises the central rise portion having one or more grooves and/or being of a lesser thickness relative to the first distal end portion and/or the second distal end portion.

6. The mouthpiece of claim 1, wherein the at least one flexibility-enhancing feature comprises a first corrugated section extending from the central rise portion towards or to the first distal end portion, and a second corrugated section extending from the central rise portion towards or to the second distal end portion.

7. The mouthpiece of claim 1, wherein the at least one flexibility-enhancing feature comprises a thermally softening material disposed on or within the first distal end portion and/or the second distal end portion, wherein the thermally softening material is configured to soften and increase flexibility of the body upon heating.

8. The mouthpiece of claim 1, wherein the at least one flexibility-enhancing features comprises at least two perforations positioned at opposite sides of the central rise portion.

9. The mouthpiece of any of the previous claims, wherein the central rise portion is configured to allow an end of the first distal end portion and/or an end of the second distal end portion to flex at least 0.5 mm, 1 mm, 2 mm, 3 mm, 4 mm, or 5 mm relative to a resting position of the end of the first distal end portion and/or of the end of the second distal end portion.

10. The mouthpiece of any of the preceding claims, wherein the top level and bottom level further comprise edges extending from the inner border and/or outer border.

11. The mouthpiece of claim 10, wherein at least some of the edges form an angle of 10 to 55 ° either upwards or downwards relative to the planar surface disposed between the inner and outer border of each level.

12. The mouthpiece of claim 10 or 11, wherein at least some of the edges are tilted so as to form a slope towards the planar surface disposed between the inner and outer border of each level.

13. The mouthpiece of any of the preceding claims, wherein the body comprises a heat conducting material having a thermal conductivity of greater than 0.5 W/mK, for example greater than 1 W/mK, in particular 1.1 to 25 W/mK for polymer materials and 100 to 500 W/mK for metal materials.

14. The mouthpiece of any of the preceding claims, wherein the body and the light sources are at least partially encapsulated by an encasing having the shape of a dental arch.

15. The mouthpiece of any of the preceding claims, wherein the body comprises an extended portion which defines a cooling tray configured to conduct heat away from the arch.

16. The mouthpiece of any of the preceding claims, wherein the light sources are capable of emitting light at an average power density of 30 to 1000 mW/cm² towards intraoral surfaces of the user.

17. A method for adjusting the fit of an intraoral mouthpiece for an individual, the method comprising:
- selecting a base size of an intraoral mouthpiece based on a size of the individual's dental arch,
- adjusting, based on a shape of the individual's dental arch, a form or configuration of the intraoral mouthpiece to achieve desired light distribution and comfort during treatment,
wherein the adjusting comprises reforming the intraoral mouthpiece over an adjustable forming jⁱg.

18. The method according to claim 17, wherein the intraoral mouthpiece comprises a first distal end portion and a second distal end portion, each end portion having an end, and wherein the reforming comprises moving the end of the first distal end portion and/or the end of the second distal end portion inwards or outwards of an arch of the intraoral mouthpiece.

19. The method according to claim 17 or 18, wherein the adjusting further comprises heating the intraoral mouthpiece prior to the reforming.

20. The method according to any one of claims 17-19, further comprising cooling the intraoral mouthpiece, allowing the intraoral mouthpiece to set in a desired position for the individual.

21. A method of treatment of teeth or oral tissue of a user, comprising
- providing a mouthpiece according to any one of claims 1 to 16;
- placing the mouthpiece in the user's mouth;
- supplying the light sources with electric power for generating light; and
- directing the light towards the user's teeth or intraoral issue to achieve photodynamic treatment of the teeth and/or oral issue.

22. A kit comprising a mouthpiece according to any of the previous claims and an intraoral agent selected from the group consisting of antioxidants, photosensitizers and potentiating agents, and combinations thereof.

23. The kit according to claim 22, wherein the photosensitizer is selected from the group consisting of Hypericin, curcumin, phenalenone derivatives, Cercosporin, psoralen, xanthotoxin, Angelicin, alpha-Terthienyl, Phenylthepatriyne, THC, Cannabidiol (CBD). Synthetic photosensitizers include the following: RB (Rose Bengal), MB, Porphyrin derivatives, Curcumin derivatives, Methylene Blue, Indocynine Green, Erythosine, Phenalenone derivatives, Fullerene derivatives, Xanthene derivates, optionally together with a pigment.
